(19) **Europäisches Patentamt / European Patent Office / Office européen des brevets**

(11) **EP 4 643 870 A1**

(12) **EUROPEAN PATENT APPLICATION**
published in accordance with Art. 153(4) EPC

(43) Date of publication:
05.11.2025 Bulletin 2025/45

(21) Application number: 23912131.2

(22) Date of filing: 26.12.2023

(51) International Patent Classification (IPC):
$A61K\ 38/16^{(2006.01)}$    $A61K\ 48/00^{(2006.01)}$
$A61P\ 25/00^{(2006.01)}$    $C07K\ 14/00^{(2006.01)}$
$C12N\ 1/15^{(2006.01)}$    $C12N\ 1/19^{(2006.01)}$
$C12N\ 1/21^{(2006.01)}$    $C12N\ 5/10^{(2006.01)}$
$C12N\ 15/09^{(2006.01)}$    $C12N\ 15/63^{(2006.01)}$

(52) Cooperative Patent Classification (CPC):
A61K 38/16; A61K 48/00; A61P 25/00;
C07K 14/00; C12N 5/10; C12N 15/09; C12N 15/63;
C12N 15/74; C12N 15/80; C12N 15/81

(86) International application number:
PCT/JP2023/046633

(87) International publication number:
WO 2024/143352 (04.07.2024 Gazette 2024/27)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 27.12.2022 JP 2022209604

(71) Applicants:
• Stemrim Inc.
Ibaraki-shi, Osaka 567-0085 (JP)
• The University of Osaka
Suita-shi, Osaka 565-0871 (JP)

(72) Inventors:
• TAMAI, Katsuto
Suita-shi, Osaka 565-0871 (JP)
• SHIMBO, Takashi
Suita-shi, Osaka 565-0871 (JP)
• YAMAZAKI, Takehiko
Ibaraki-shi, Osaka 567-0085 (JP)

(74) Representative: Moré, Solveig Helga et al
Kroher Strobel
Rechts- und Patentanwälte PartmbB
Bavariaring 20
80336 München (DE)

Remarks:
The complete document including Reference Table(s) and the Sequence Listing(s) can be downloaded from the EPO website

(54) **PEPTIDE AND USE THEREOF**

(57) [Problem] An objective of the present application is to provide a peptide and use thereof.

[Solution] Based on the results of their original studies conducted to date, the inventors designed an artificial sequence peptide consisting of the amino acid sequence set forth in SEQ ID NO: 1 and found that the artificial sequence peptide shows therapeutic effects on traumatic brain injury. Based on these findings, the present application provides a peptide, such as an artificial sequence peptide consisting of the amino acid sequence set forth in SEQ ID NO: 1 and use thereof.

EP 4 643 870 A1

[Fig. 1-1]

## Number of Slips

*: Vehicle vs Artificial sequence peptide
* p<0.05
Day 14: p=0.0292

[Fig. 1-2]

## Latency to Traverse Beam

*: Vehicle vs Artificial sequence peptide
*:p<0.05, **:p<0.01
Day 8 : p = 0.0359
Day 14 : p = 0.0049

**Description**

TECHNICAL FIELD

The present application relates to a peptide and use thereof.
This application claims priority to Japanese Patent Application No. 2022-209604, filed on December 27, 2022, the entire contents of which are incorporated herein by reference.

BACKGROUND ART

Traumatic Brain Injury (TBI) refers to damage to the brain caused by external mechanical forces and is recognized as one of the leading causes of morbidity and mortality worldwide. Nevertheless, currently, treatment options for TBI are limited and consist mainly of medical support and rehabilitation, with no effective pharmacological approach. Therefore, the development of effective drugs for the treatment of TBI is desirable.

SUMMARY OF THE INVENTION

PROBLEMS TO BE SOLVED BY THE INVENTION

An objective of the present application is to provide a peptide and use thereof.

MEANS FOR SOLVING THE PROBLEMS

Based on the results of their original studies conducted to date, the inventors designed an artificial sequence peptide consisting of the amino acid sequence set forth in SEQ ID NO: 1 and found that the artificial sequence peptide shows therapeutic effects on traumatic brain injury. Based on these findings, the present application provides a peptide, such as an artificial sequence peptide consisting of the amino acid sequence set forth in SEQ ID NO: 1 and use thereof.
That is, the present disclosure provides following embodiments.

[1] A pharmaceutical composition for preventing or treating brain injury, which comprises a peptide selected from the following:

(a) a peptide comprising the amino acid sequence set forth in SEQ ID NO: 1;
(b) a peptide comprising an amino acid sequence in which one or a plurality of amino acids have been substituted, deleted, inserted, or added in the amino acid sequence set forth in SEQ ID NO: 1; and
(c) a peptide comprising an amino acid sequence having about 80% or more sequence identity with the amino acid sequence set forth in SEQ ID NO: 1.

[2] The pharmaceutical composition according to [1], wherein the brain injury is a focal or diffuse brain injury.
[3] The pharmaceutical composition according to [1], wherein the brain injury is selected from cerebral contusion, epidural hematoma, subdural hematoma, intracerebral hematoma, cerebral concussion, diffuse axonal injury, and subarachnoid hemorrhage.
[4] The pharmaceutical composition according to [1], wherein the brain injury is traumatic brain injury.
[5] A peptide selected from:

(a) a peptide comprising the amino acid sequence set forth in SEQ ID NO: 1;
(b) a peptide comprising an amino acid sequence in which one or a plurality of amino acids have been substituted, deleted, inserted, or added in the amino acid sequence set forth in SEQ ID NO: 1; and
(c) a peptide comprising an amino acid sequence having about 80% or more sequence identity with the amino acid sequence set forth in SEQ ID NO: 1.

[6] A composition comprising the peptide according to [5].
[7] A pharmaceutical composition comprising the peptide according to item 5.
[8] A nucleic acid encoding the peptide according to [5].
[9] A vector comprising the nucleic acid according to [8].
[10] A host cell comprising the nucleic acid according to [8] or the vector according to [9].
[11] A composition comprising the nucleic acid according to [8] or the vector according to [9].
[12] A pharmaceutical composition comprising the nucleic acid according to [8] or the vector according to [9].

[A1] A method of preventing or treating brain injury, comprising a step of administering to a subject an effective amount of a peptide selected from the following:

(a) a peptide comprising the amino acid sequence set forth in SEQ ID NO: 1;
(b) a peptide comprising an amino acid sequence in which one or a plurality of amino acids have been substituted, deleted, inserted, or added in the amino acid sequence set forth in SEQ ID NO: 1; and
(c) a peptide comprising an amino acid sequence having about 80% or more sequence identity with the amino acid sequence set forth in SEQ ID NO: 1.

[A2] The method according to [A1], wherein the brain injury is a focal or diffuse brain injury.
[A3] The method according to [A1], wherein the brain injury is selected from cerebral contusion, epidural hematoma, subdural hematoma, intracerebral hematoma, cerebral concussion, diffuse axonal injury, and subarachnoid hemorrhage.
[A4] The method according to [A1], wherein the brain injury is traumatic brain injury.
[A5] A method of preventing or treating a disease or pathological condition, comprising a step of administering to a subject an effective amount of the peptide set forth in [A1].
[A6] A method of preventing or treating a disease or pathological condition, comprising a step of administering to a subject a nucleic acid encoding the peptide set forth in [A1] or a vector comprising said nucleic acid.
[B1] A peptide for use in preventing or treating brain injury, selected from the following:

(a) a peptide comprising the amino acid sequence set forth in SEQ ID NO: 1;
(b) a peptide comprising an amino acid sequence in which one or a plurality of amino acids have been substituted, deleted, inserted, or added in the amino acid sequence set forth in SEQ ID NO: 1; and
(c) a peptide comprising an amino acid sequence having about 80% or more sequence identity with the amino acid sequence set forth in SEQ ID NO: 1.

[B2] The peptide for use according to [B1], wherein the brain injury is a focal or diffuse brain injury.
[B3] The peptide for use according to [B1], wherein the brain injury is selected from cerebral contusion, epidural hematoma, subdural hematoma, intracerebral hematoma, cerebral concussion, diffuse axonal injury, and subarachnoid hemorrhage.
[B4] The peptide for use according to [B1], wherein the brain injury is traumatic brain injury.
[B5] The peptide set forth in [B1], wherein the peptide is for use in preventing or treating a disease or pathological condition.
[B6] A nucleic acid encoding the peptide set forth in [B1] for use in preventing or treating a disease or pathological condition.
[B7] A vector comprising a nucleic acid encoding the peptide set forth in [B1] for use in preventing or treating a disease or pathological condition.
[C1] Use of a peptide for manufacture of a medicament for preventing or treating brain injury, wherein the peptide is selected from the following:

(a) a peptide comprising the amino acid sequence set forth in SEQ ID NO: 1;
(b) a peptide comprising an amino acid sequence in which one or a plurality of amino acids have been substituted, deleted, inserted, or added in the amino acid sequence set forth in SEQ ID NO: 1; and
(c) a peptide comprising an amino acid sequence having about 80% or more sequence identity with the amino acid sequence set forth in SEQ ID NO: 1.

[C2] The use according to [C1], wherein the brain injury is a focal or diffuse brain injury.
[C3] The use according to [C1], wherein the brain injury is selected from cerebral contusion, epidural hematoma, subdural hematoma, intracerebral hematoma, cerebral concussion, diffuse axonal injury, and subarachnoid hemorrhage.
[C4] The use according to [C1], wherein the brain injury is traumatic brain injury.
[C5] Use of the peptide set forth in [C1] for manufacture of a medicament for preventing or treating a disease or pathological condition.
[C6] Use of a nucleic acid encoding the peptide set forth in [C1] or a vector comprising said nucleic acid for manufacture of a medicament for preventing or treating a disease or pathological condition.
[C7] Use of a vector comprising a nucleic acid encoding the peptide set forth in [C1] for manufacture of a medicament for preventing or treating a disease or pathological condition.

BRIEF DESCRIPTION OF THE DRAWINGS

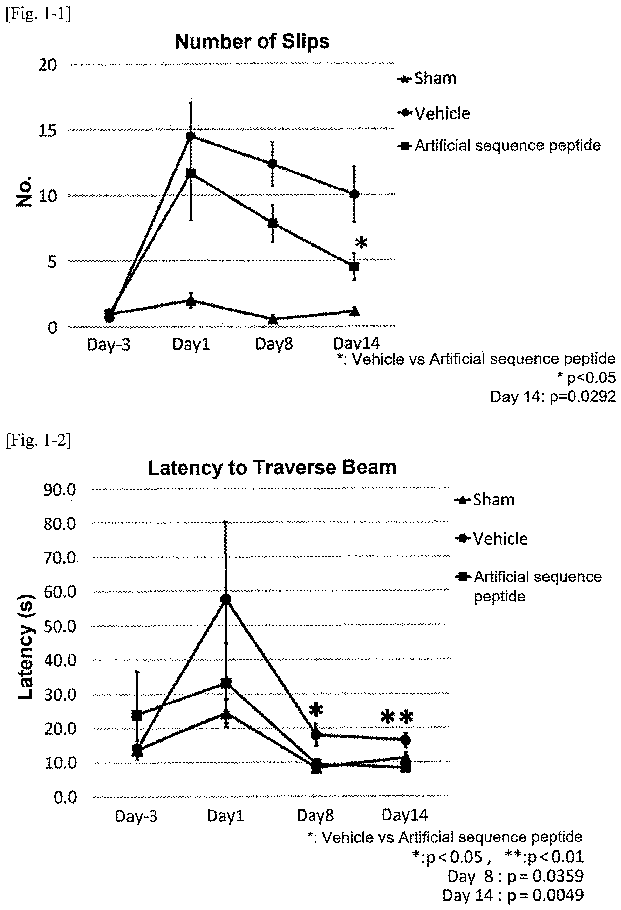

[Fig. 1-1] (Acute stage) A graph showing the change over time in the number of slips in the beam balance test in a mouse model of traumatic brain injury. * $p < 0.05$.

[Fig. 1-2] (Acute stage) A graph showing the change over time in latency to traverse the beam in the beam balance test in a mouse model of traumatic brain injury. * $p < 0.05$, ** $p < 0.01$.

[Fig. 2-1] (Subacute stage) A graph showing the change over time in the number of slips in the beam balance test in a mouse model of traumatic brain injury. ** $p < 0.01$.

[Fig. 2-2] (Subacute stage) A graph showing the change over time in latency to traverse the beam in the beam balance test in a mouse model of traumatic brain injury. * $p < 0.05$, ** $p < 0.01$.

[Fig. 3-1] (Chronic stage) A graph showing the change over time in the number of slips in the beam balance test in a mouse model of traumatic brain injury. *** $p < 0.001$.

[Fig. 3-2] (Chronic stage) A graph showing the change over time in latency to traverse the beam in the beam balance test in a mouse model of traumatic brain injury. ** $p < 0.01$, *** $p < 0.001$.

[Fig. 4] (Chronic stage) A graph showing the change over time in time spent on the rotor during the rotarod test in a mouse model of traumatic brain injury. ** $p < 0.01$, *** $p < 0.001$.

[Fig. 5] A photograph of the apparatus used for the Y-maze test. Three arms (A, B, C) of equal size are connected in a Y-shape at 120°.

[Fig. 6] (Severe) A graph showing the change over time in alternation (%) in the Y-maze test in a mouse model of traumatic brain injury (referred to as "Y-maze correct rate").

[Fig. 7-1] (Subacute stage) A graph showing the change over time in the number of slips in the beam balance test in a mouse model of traumatic brain injury. * $p < 0.05$, ** $p < 0.01$.

[Fig. 7-2] (Subacute stage) A graph showing the change over time in latency to traverse the beam in the beam balance test in a mouse model of traumatic brain injury. * $p < 0.05$, ** $p < 0.01$.

DETAILED DESCRIPTION

The present application provides a certain peptide, a composition comprising the peptide, a pharmaceutical composition comprising the peptide, a nucleic acid encoding the peptide, a composition comprising the nucleic acid, and a pharmaceutical composition comprising the nucleic acid.

As used herein, "a peptide" includes "an artificial sequence peptide". As used herein, "an artificial sequence peptide" refers to a peptide having a non-naturally occurring amino acid sequence. As used herein, "an artificial sequence peptide" is also referred to simply as "an artificial peptide".

The peptide of the present application includes, for example, a peptide selected from the followings:

(a) a peptide comprising the amino acid sequence set forth in SEQ ID NO: 1;
(b) a peptide comprising an amino acid sequence in which one or a plurality of amino acids have been substituted, deleted, inserted, or added in the amino acid sequence set forth in SEQ ID NO: 1; and
(c) a peptide comprising an amino acid sequence having about 80% or more sequence identity with the amino acid sequence set forth in SEQ ID NO: 1.

The peptides listed in (a) through (c) above include a peptide selected from the following:

(1) a peptide consisting of the amino acid sequence set forth in SEQ ID NO: 1;
(2) a peptide consisting of an amino acid sequence in which one or a plurality of amino acids have been substituted, deleted, inserted, or added in the amino acid sequence set forth in SEQ ID NO: 1;
(3) a peptide consisting of an amino acid sequence having about 80% or more sequence identity with the amino acid sequence set forth in SEQ ID NO: 1;
(4) a peptide consisting of a portion of the amino acid sequence set forth in SEQ ID NO: 1;
(5) a peptide encoded by a DNA that hybridizes under a stringent condition with a DNA consisting of the nucleotide sequence set forth in SEQ ID NO: 2; and
(6) a peptide encoded by a DNA consisting of the nucleotide sequence set forth in SEQ ID NO: 2.

The peptides described in (a) through (c) and (2) through (6) above may be a peptide having an effect of improving motor function or restoring memory function in brain injury. The peptides described in (a) through (c) and (2) through (6) above may be a peptide functionally equivalent to a peptide comprising the amino acid sequence set forth in SEQ ID NO: 1 ("functional equivalent"), and examples of the peptide include peptides comprising the amino acid sequences set forth in SEQ ID NOs: 3, 5, 7, 9, 11, 13, 15, 17, 19, 21, 23, 25, and 27, respectively. As used herein, "functionally equivalent" means that the effect of improving motor function or restoring memory function in brain injury is equivalent. The effect can be evaluated, for example, in the experimental system described in the working examples of the present application.

As used herein, "plurality" includes, for example, two to six, two to five, two to five, two to four, two to three, or two.

As used herein, "about 80% or more" includes, for example, about 80% or more, about 85% or more, about 90% or more, about 91% or more, about 92% or more, about 93% or more, about 94% or more, about 95% or more, about 96% or more, about 97% or more, about 98% or more, or about 99% or more.

As used herein, "a peptide comprising the amino acid sequence set forth in SEQ ID NO: 1" includes "a peptide consisting of the amino acid sequence set forth in SEQ ID NO: 1". As used herein, "a peptide comprising the amino acid sequence set forth in SEQ ID NO: 1" is 30 to X (i.e., not less than 30 and not more than X) or 31 to X (i.e., not less than 31 and not more than X) amino acids in length. For example, X is selected from, but is not limited to, 100, 95, 90, 85, 80, 75, 70, 65, 60, 55, 50, 45, 40, 39, 38, 37, 36, 35, 34, 33, 32, and 31.

As used herein, "a peptide comprising the amino acid sequence set forth in SEQ ID NO: 1" includes a peptide consisting of an amino acid sequence having one or a plurality of amino acid additions at the N- and/or C-terminal of the amino acid sequence set forth in SEQ ID NO: 1. As used herein, "and/or" is used interchangeably with "both or either".

As used herein, "a peptide consisting of a portion of the amino acid sequence set forth in SEQ ID NO: 1" is from Y to 29 (i.e., not less than Y and not more than 29) amino acids in length. For example, Y is selected from, but is not limited to, 10, 15, 20, 21, 22, 23, 24, 25, 26, 27, and 28.

As used herein, "a stringent condition" may refer to, for example, a condition such as hybridization with 6 x SSC and 40% formamide, at 25°C and washing with 1 x SSC, at 55°C. Stringency depends on conditions such as salt concentration, formamide concentration, or temperature, but a person skilled in the art can set these conditions to provide the required stringency.

When hybridization is performed under a stringent condition, DNAs with a highly homologous nucleotide sequence are selected. Consequently, the peptides isolated are likely to contain peptides that are functionally equivalent to the peptide consisting of the amino acid sequence set forth in SEQ ID NO: 1. The highly homologous nucleotide sequences may have, for example, about 60% or more, about 70% or more, or about 80% or more identity.

The nucleotide sequences set forth in SEQ ID NOs: 2, 4, 6, 8, 10, 12, 14, 16, 18, 20, 22, 24, 26, and 28 are examples of nucleotide sequences of DNAs encoding the peptides set forth in SEQ ID NOs: 1, 3, 5, 7, 9, 11, 13, 15, 17, 19, 21, 23, 25, and 27, respectively. Other DNA sequences encoding the peptide of the present application may be generated by the method of converting the amino acid residues of the peptides into their corresponding codons using a codon table known to those skilled in the art (reverse translation). Reverse translation may optionally be performed using various software (including programs, algorithms, etc.) that have been developed for amino acid and nucleic acid sequence analysis.

The peptide of the present application may be obtained as a recombinant by incorporating the DNA encoding them into a suitable expression system, or by synthesizing them artificially. Thus, the peptide of the present application includes peptides produced using cells, as well as those artificially synthesized (i.e., synthetic peptides).

To obtain the peptide of the present application by genetic engineering, the DNA encoding the peptide may be incorporated into an appropriate expression system and expressed.

Suitable hosts for the present application include, but are not limited to, prokaryotic and eukaryotic cells. The hosts above also include, but are not limited to, bacteria (e.g., E. coli), yeast, animal cells (e.g., mammalian cells such as HEK293 cells and CHO cells, and insect cells such as silkworm cells), and plant cells.

For example, a host/vector system suitable for the present application may include the pGEX expression vector and E. coli. Since pGEX can express an exogenous gene as a fusion protein with glutathione S-transferase (GST) (Gene, 67:31-40, 1988), pGEX containing a DNA encoding the peptide of the present application is introduced into an E. coli strain such as BL21 by heat shock and, after a suitable incubation period, isopropylthio-β-D-galactoside (IPTG) is added to induce expression of the GST fusion peptide. Since the GST of the present application binds to Glutathione Sepharose 4B, the expression product can be easily separated and purified by affinity chromatography.

Other suitable host/vector systems may also be employed to obtain the recombinant peptides of the present application. For example, when the host is bacteria, expression vectors for fusion proteins using tags or the like are commercially available. The recombinant peptides of the present application also include those to which a tag or a portion of a tag is attached.

The tag to be attached to the peptide of the present application is not particularly limited unless it affects the activity of the peptide. Examples of tags include a histidine tag (e.g., 6 x His, 10 x His), HA tag, FLAG tag, GST tag, T7 tag, HSV tag, E tag, lck tag, and B-tag.

Among yeast, it is known that Pichia sp. yeast is effective in expressing proteins with glycans. For the purpose of adding a glycan, a baculovirus vector expression system using an insect cell as a host is also useful (Bio/Technology, 6:47-55, 1988). In addition, mammalian cells have been used as hosts for transfection with vectors containing CMV, RSV, or SV40 promoters, and any of these host/vector systems can serve as an expression system for the peptide of the present application. Genes may also be introduced using vectors including, but not limited to, a plasmid vector or a viral vector such as retrovirus vector, lentivirus vector, adenovirus vector, adeno-associated virus vector, Sendai virus vector, Sendai virus envelope vector, and papillomavirus vector. The vector may contain a promoter DNA sequence that effectively induces gene expression, a factor that controls gene expression, and a molecule necessary to maintain DNA stability.

The resulting peptide can be isolated from inside or outside the host cells (e.g., from the culture medium) and purified to be substantially pure and homogeneous. The separation and purification of the peptide can be performed by any method commonly employed in peptide purification and is not limited to a specific method. For example, the peptide can be separated and purified appropriately selecting and combining techniques such as chromatographic columns, filters, ultrafiltration, salt precipitation, solvent precipitation, solvent extraction, distillation, immunoprecipitation, SDS-polyacrylamide gel electrophoresis, isoelectric focusing, dialysis, and recrystallization.

Chromatography includes, for example, affinity chromatography, ion exchange chromatography, hydrophobic interaction chromatography, gel filtration, reversed-phase chromatography, and adsorption chromatography (Marshak et al., Strategies for Protein Purification and Characterization: A Laboratory Course Manual, Cold Spring Harbor Lab Press, 1996). Chromatography can be performed using liquid chromatography, e.g., HPLC or FPLC.

Preferably, the peptide of the present application is a substantially purified peptide. As used herein, "substantially purified" means that the degree of purification of the peptide of the present application (the percentage of the peptide in the total protein component) is 50% or more, 60% or more, 70% or more, 80% or more, 90% or more, or 95% or more, 100% or near 100%. The upper limit of "near 100%" depends on the purification and analytical techniques of a person skilled in the art and may be, for example, 99.999%, 99.99%, 99.9%, or 99%.

The substantially purified peptide may be any peptide purified by any purification method if the peptide is purified to the degree described above. Examples of substantially purified peptides include peptides purified by any or a combination of methods as listed above including, but not limited to, chromatographic columns, filters, ultrafiltration, salt precipitation, solvent precipitation, solvent extraction, distillation, immunoprecipitation, SDS-polyacrylamide gel electrophoresis, isoelectric focusing, dialysis, and recrystallization.

Alternatively, the peptide of the present application can be synthesized artificially. The peptide synthesis method of the present application allows for the chemical synthesis of peptides, for example, using liquid-phase or solid-phase peptide synthesis. Solid-phase peptide synthesis is a commonly used method for the chemical synthesis of peptides. This method uses polystyrene polymer gel beads with a diameter of about 0.1 mm as the solid phase, the surface of which is modified with amino groups. Then, amino acid chains are sequentially elongated via dehydration reactions. Once the desired peptide sequence has been synthesized, it is cleaved from the solid phase surface to yield the product. Solid-phase synthesis also allows the synthesis of ribosomal peptides that are difficult to synthesize in bacteria, the introduction of non-natural amino acids such as D-amino acids or amino acids substituted with a stable isotope (e.g., $^2$H, $^{13}$C, or $^{15}$N), the introduction of amino acids substituted with a heavy atom (e.g., selenoamino acids such as selenomethionine), and the modification of the peptide and protein backbones. When synthesizing a long peptide chain of 70 to 100 or more amino acids in the solid-phase synthesis, the native chemical ligation method can be used to join two peptide chains into one long peptide chain. The peptide in the present application includes both unmodified and modified peptides.

The nucleic acid of the present application includes DNA and RNA. The RNA includes, but is not limited to, mRNA. Preferably, the nucleic acid of the present application is a substantially purified nucleic acid. The nucleic acid of the present application includes both unmodified and modified nucleic acids.

In the present application, one or more peptides or one or more nucleic acids may be included in a composition. The peptide, nucleic acid, or vector comprising the peptide of the present application may be dissolved or suspended in a solvent in the composition. The solvent includes, but are not limited to, water, saline, or a buffer. The buffer includes, but are not limited to, citrate buffer, acetate buffer, or phosphate buffer.

The composition of the present application may be used as a pharmaceutical composition or a reagent composition. As used herein, the term "pharmaceutical composition" is used interchangeably with "medicament," "pharmaceutical preparation" or "medicinal composition", and the term "reagent composition" is used interchangeably with "reagent".

As used herein, the term "pharmaceutical composition comprising (an agent)" is used interchangeably with "pharmaceutical composition comprising (an agent) as an active ingredient".

The peptide or the nucleic acid of the present application may be used in preventing or treating traumatic brain injury. In traumatic brain injury, physical damage to the head results in destruction of brain parenchymal tissue, disruption of blood vessels in the brain parenchyma, and disruption of blood vessels on the brain surface. In addition, hemorrhages in the peri-brain region (e.g., dura and arachnoid), brain parenchyma, and brain surface result in hematomas and cause damage by compressing the brain. Considering the etiology and/or pathogenesis as described above, the peptide of the present application may be used not only for the treatment of traumatic brain injury, but also for the treatment or prevention of brain injury.

In the present application, brain injuries include, but are not limited to, focal brain injury, diffuse brain injury, traumatic brain injury, brain contusion, epidural hematoma, subdural hematoma, intracerebral hematoma, cerebral concussion, diffuse axonal injury, and subarachnoid hemorrhage. The diseases listed above include traumatic diseases or non-traumatic (e.g., intrinsic) diseases. The diseases listed above include acute, subacute, or chronic stage of the diseases.

The diagnostic criteria for these diseases are well-known. The prevention and treatment of the diseases can be evaluated based on these criteria.

The peptide or the nucleic acid of the present application may also be used in basic and clinical research, for example. The present application also provides the use of the peptide of the present application in manufacture of a medicament or reagent for basic or clinical research.

The subject of the present application is not particularly limited, and includes, for example, mammals, birds, and fish. The mammals include human or non-human animals including, but not limited to, human, mouse, rat, monkey, pig, dog, rabbit, hamster, guinea pig, horse, sheep, and whale. As used herein, the term "subject" is used interchangeably with "patient," "individual", or "animal".

An effective amount of the peptide of the present application or a pharmaceutical composition comprising the same, the nucleic acid of the present application or a pharmaceutical composition comprising the same, or the vector of the present application or a pharmaceutical composition comprising the same (hereinafter referred to as "pharmaceutical composition or the like") is administered to a subject to treat the disease or symptom described herein.

An effective amount in the present application refers to an amount sufficient for the prevention or treatment of the disease or pathological condition described herein. The treatment includes, but is not limited to, alleviation, delay, inhibition, amelioration, remission, cure, and complete cure. The prevention includes, but is not limited to, alleviation, delay, and inhibition.

In the present application, the site of administration of the pharmaceutical composition or the like is not limited. The effect of the pharmaceutical composition or the like can be achieved when it is administered to a site, such as a site at or near the site where a symptom of a disease or pathological condition appears, a site different from the site at or near the site where a symptom of a disease or pathological condition appears (a site other than that site), a site distant from the site where a symptom of a disease or pathological condition appears, a site distal to the site where a symptom of a disease or pathological condition appears, or a site distal and heterologous to the site where a symptom of a disease or pathological condition appears, or any other sites.

In the present application, the effect of the pharmaceutical composition or the like can be achieved when it is administered to a tissue, such as a tissue different from the tissue in which a symptom of a disease or pathological condition appears, a tissue distant from the tissue in which a symptom of a disease or pathological condition appears, a tissue distal to the tissue in which a symptom of a disease or pathological condition appears, or a tissue distal and heterologous to the tissue in which a symptom of a disease or pathological condition appears, or any other tissues.

In the present application, methods of administration of the pharmaceutical composition or the like include oral or parenteral administration. Methods of parenteral administration include, but are not limited to, intravascular (e.g., intraarterial or intravenous), intramuscular, subcutaneous, intradermal, intraperitoneal, intranasal, transpulmonary, and percutaneous administration. The pharmaceutical composition or the like may be administered systemically or locally (e.g., subcutaneously, intradermally, on the skin surface, on the ocular or eyelid conjunctiva, nasal mucosa, oral and digestive tract mucosa, vaginal and uterine mucosa, or at the site of injury) by injection, e.g., intravenous, intramuscular, intraperitoneal, or subcutaneous injection. In other words, the methods of administration of the pharmaceutical composition or the like include systemic and topical administration.

The peptide of the present application may also be used in the form of a nucleic acid encoding the peptide, a vector comprising the nucleic acid, a cell that secretes the peptide, or a gene therapy vector containing a DNA encoding the peptide.

The peptide of the present application may be in the form of a pharmaceutically acceptable salt. Pharmaceutically acceptable salts include, but are not limited to, hydrochloride, acetate, and trifluoroacetate. The peptide of the present application may be in the form of a solvate of the peptide or a solvate of a pharmaceutically acceptable salt of the peptide. A solvate is a substance in which any number of solvent molecules are coordinated to a solute molecule, including, but not limited to, hydrates. The peptide of the present application may also be any optical or structural isomer.

The nucleic acid of the present application may be in the form of a pharmaceutically acceptable salt. Pharmaceutically acceptable salts include, but are not limited to, an alkali metal salt, alkaline earth metal salt, metal salt, inorganic salt, inorganic acid salt, amine salt, lower alkane sulfonate, aryl sulfonate, organic salt, organic acid salt, and amino acid salt.

The nucleic acid of the present application may be administered to a subject using, for example, a liposome, retrovirus, adenovirus, adeno-associated virus, and vaccinia vector.

The method of administration may be selected depending on the patient's age and symptoms. When administering the pharmaceutical composition or the like of the present application, the dosage may be selected, for example, in the range of 0.0000001 mg to 1000 mg per kg body weight per administration. Alternatively, the dosage may be selected, for example, in the range of 0.00001 to 100000 mg/body per patient. When administering cells that secrete the peptide of the present application or a gene therapy vector containing a DNA encoding the peptide, the administration may be performed such that the amount of the peptide falls within the range described above. However, possible dosages of the pharmaceutical composition of the present application are not limited to those described above.

The pharmaceutical composition of the present application can be formulated using conventional methods, such as those described in Remington's Pharmaceutical Science, latest edition (Mark Publishing Company, Easton, U.S.A.). The pharmaceutical composition may further contain a pharmaceutically acceptable carrier or additive. Examples of the carrier or the additive include a surfactant, an excipient, a colorant, a flavoring agent, a preservative, a stabilizer, a buffer, a suspending agent, an isotonic agent, a binder, a disintegrant, a lubricant, a fluidity promoter, a flavor modifier, but are not limited to these, and other conventionally used carriers and additives. Specific examples thereof include light anhydrous silicic acid, lactose, crystalline cellulose, mannitol, starch, carmellose calcium, carmellose sodium, hydroxypropyl cellulose, hydroxypropyl methylcellulose, polyvinyl acetal diethylamino acetate, polyvinylpyrrolidone, gelatin, medium-chain fatty acid triglyceride, polyoxyethylene hydrogenated castor oil 60, sucrose, carboxymethyl cellulose, corn starch, inorganic salts, glucose, and the like.

It should be noted that all the prior art literatures cited herein are incorporated herein as references.

The present invention is further exemplified by, but not limited to, the following examples.

EXAMPLES

Example 1

Evaluation of the Efficacy of Artificial Sequence Peptide for Acute Traumatic Brain Injury

(1) Materials and methods

i) Production of peptide

An artificial sequence peptide consisting of the amino acid sequence set forth in SEQ ID NO: 1 was chemically synthesized (the resulting peptide was in the form of a trifluoroacetic acid or hydrochloric acid (TFA or HCl) salt).

ii) Preparation of a mouse model of traumatic brain injury (TBI)

Male C57BL/6J Jcl mice (8 weeks old) were purchased from CLEA Japan and then acclimated for five days or more. Prior to surgery, mice were anesthetized with isoflurane inhalation, the top of the mouse head was shaved, and then the mouse head was fixed in a stereotaxic head frame. After disinfecting the parietal skin with 70% ethanol, a median incision was made in the parietal region using a scalpel. The fascia was removed with a cotton swab to expose the skull, the bregma was identified, a mark was made on the skull 2 mm to the right lateral and 2 mm caudal to the bregma, a 3 mm diameter circle was drawn centered on this mark, the bone was cut along the circle with a dental drill, and the skull fragment was slowly removed with forceps once the dura mater was reached. If bleeding was present, a cotton swab was used to wipe and stop the bleeding. An impactor (Leica) was then used to create a traumatic contusion perpendicular to the cortical surface of the mouse at 2 mm diameter, 1.0 mm depth, 4.0 m/sec velocity, 0.2 seconds duration, and 10 degrees of obliquity. This procedure is widely used by researchers studying traumatic brain injury and is the most consistent and reproducible form of injury. After the injury, the mice were removed from the stereotaxic head frame and allowed to breathe. The incision was simply sutured with a clip, gentacin was applied, and then the mouse was returned to its home cage. Sham-operated (Sham) mice underwent the same surgical procedure as above, except for removal of the skull fragment followed by the creation of a traumatic contusion using an impactor. Those with ruptured or damaged dura mater were excluded during preparation and were not used in any further experiments.

iii) Administration of Peptide

Traumatic brain injury model mice prepared as described above were divided into an artificial sequence peptide-treated group (n = 6) and a control group (n = 6). The peptide (hydrochloride) (1.39 mg/kg, or approximately 0.3 $\mu$mol/kg) was administered once three hours after TBI surgery (10 mL/kg dose volume). To the control group the same volume of saline was injected via the tail vein at the same schedule as in the peptide-treated group. To sham-operated (Sham) mice (n = 6) saline was also injected via the tail vein in the same manner as the control group.

iv) Evaluation of motor function

Motor function was evaluated by a beam balance test. Specifically, a stainless-steel beam, 1 m long and 1 cm wide, was placed at a 10° slope from the front (as a starting point) to the back, and the mice were allowed to spontaneously walk across the beam. The time taken for the mouse to cross from the starting point (latency to traverse the beam) and the number of slips during this time were counted. The test was performed prior to administration of peptide, essentially once a day, once a week. Training was performed twice per mouse on two separate days prior to TBI surgery.

v) Statistical processing method

Each measurement was presented as the mean ± standard error of the mean (mean ± SEM). Comparison between the control group and the peptide-treated group was performed using the Mann-Whitney U test for the number of slips and the Student's t-test for the latency to traverse the beam. p values of $< 0.05$, $< 0.01$, or $< 0.001$ were considered statistically significant.

(2) Result

Significant reductions in the number of slips (* $p < 0.05$) and latency to traverse the beam (* $p < 0.05$, ** $p < 0.01$) were observed in the peptide-treated group compared to the control group in the beam balance test (Figs. 1-1 and 1-2). These results demonstrate the efficacy of the artificial sequence peptide in improving motor function in acute traumatic brain injury.

Example 2

Evaluation of the Efficacy of Artificial Sequence Peptide for Subacute Traumatic Brain Injury

(1) Materials and Methods

i) Preparation of a mouse model of traumatic brain injury

Traumatic brain injury model mice and sham-operated (Sham) mice were prepared in the same method as described in Example 1.

ii) Administration of Peptide

Traumatic brain injury model mice prepared as described above were divided into a peptide-treated group (n = 6) and a control group (n = 6). The peptide (trifluoroacetate) (1.77 mg/kg, or approximately 0.3 μmol/kg) was administered as a single dose (10 mL/kg dose volume) via the tail vein on the 14th day after TBI surgery. To the control group the same volume of saline was injected via the tail vein at the same schedule as in the peptide-treated group. To sham-operated (Sham) mice (n = 6) saline was also injected via the tail vein, as in the control group.

iii) Evaluation of motor function

Motor function was evaluated by a beam balance test as described in Example 1.

(2) Result

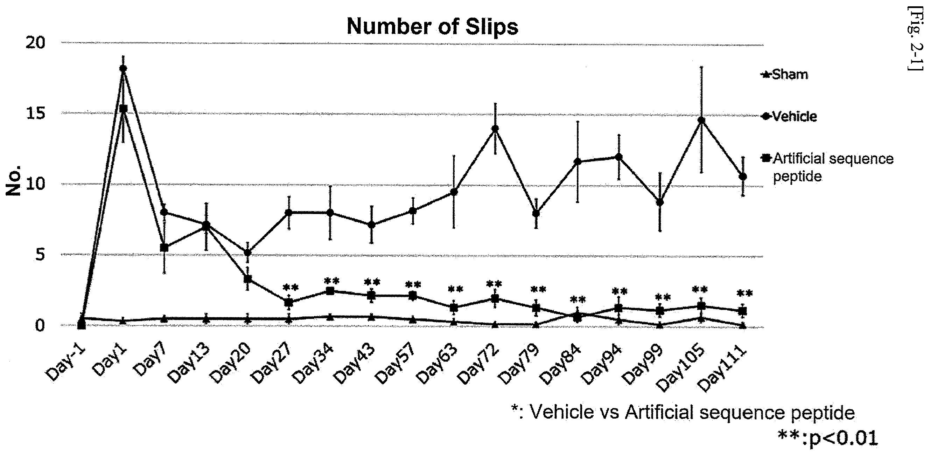

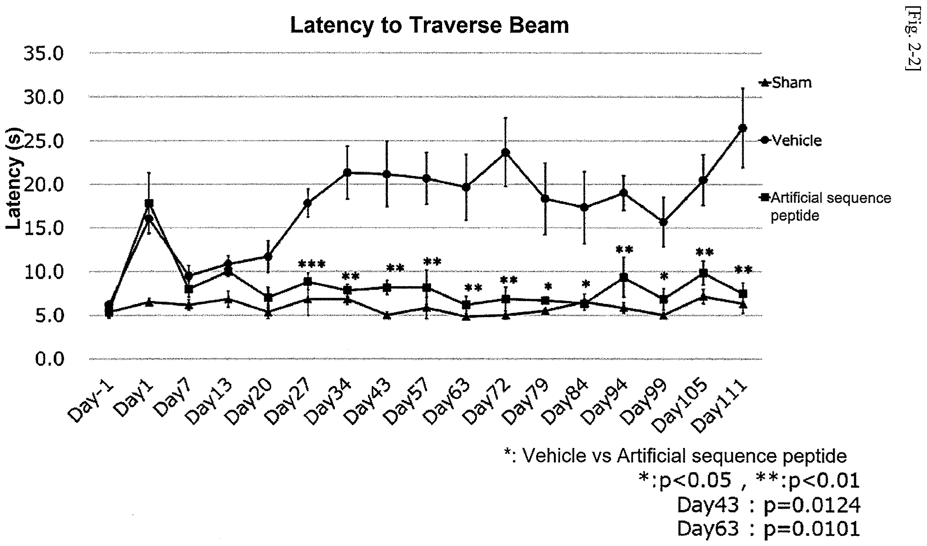

Significant reductions in the number of slips (** $p < 0.01$) and in latency to traverse the beam (* $p < 0.05$, ** $p < 0.01$) were observed in the peptide-treated group compared to the control group in the beam balance test (Figs. 2-1 and 2-2). These results demonstrate the efficacy of the artificial sequence peptide in improving motor function in subacute traumatic brain injury.

Example 3

Efficacy of Artificial Sequence Peptide in Chronic Traumatic Brain Injury

(1) Materials and Methods

i) Preparation of a mouse model of traumatic brain injury (TBI)

Traumatic brain injury model mice and sham-operated (Sham) mice were prepared by the same method as described in Example 1.

ii) Administration of Peptide

Traumatic brain injury model mice prepared as described above were divided into a peptide-treated group (n = 10) and a control group (n = 10). The peptide (trifluoroacetate) (3 mg/kg, or approximately 0.5 $\mu$mol/kg) was administered via the tail vein (10 mL/kg dose volume) for a total of 16 doses: once daily for five consecutive days starting on day 48 after TBI surgery, and then twice weekly starting the following week. To the control group the same volume of saline was injected via the tail vein at the same schedule as in the peptide-treated group. To sham-operated (Sham) mice (n = 9) saline was also injected via the tail vein, as in the control group.

iii) Evaluation of motor function

Motor function was evaluated by a beam balance test as described in Example 1. In addition, a Rotarod test was performed. Each mouse was trained at 10 rpm for 2 minutes, rested for 30 minutes, and then underwent the actual test. During the test, the Rotarod setting accelerated from 0 to 40 rpm over 5 minutes. The mice were placed on a rotor, and the time (in seconds) it took for each mouse to no longer be able to keep up with the rotor's speed was measured. The mice were pre-trained at 10 rpm for 2 minutes, once per day, for two days, starting three days before surgery.

iv) Statistical processing method

Each measurement was presented as the mean $\pm$ standard error of the mean (mean $\pm$ SEM). Comparisons between the control group and the peptide-treated group were performed using the Mann-Whitney U test for the number of slips and the Student's t-test for the latency to traverse the beam and the time spent on the rotor. p values of $< 0.05$, $< 0.01$, or $< 0.001$ were considered statistically significant.

(2) Result

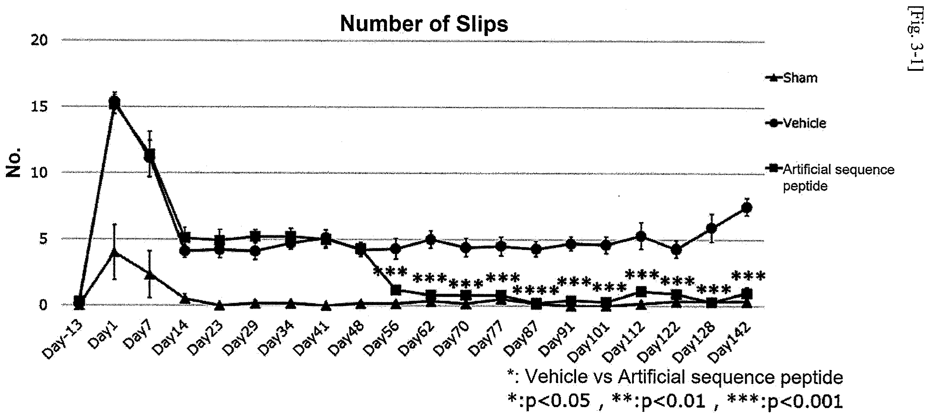

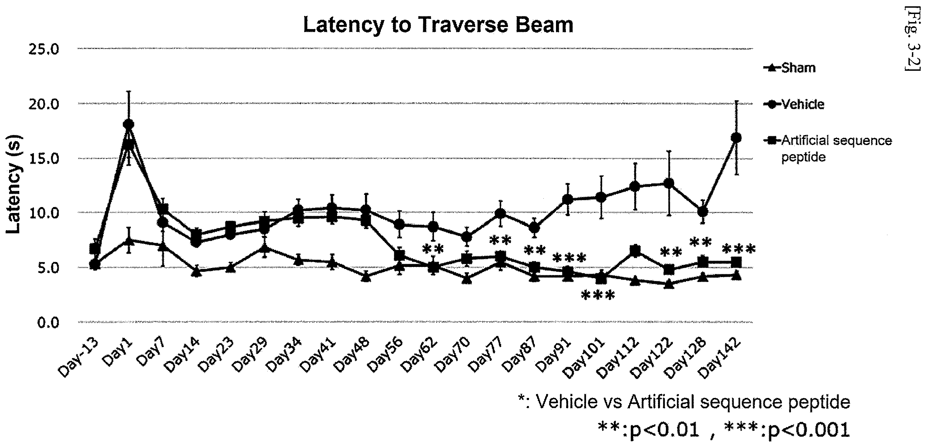

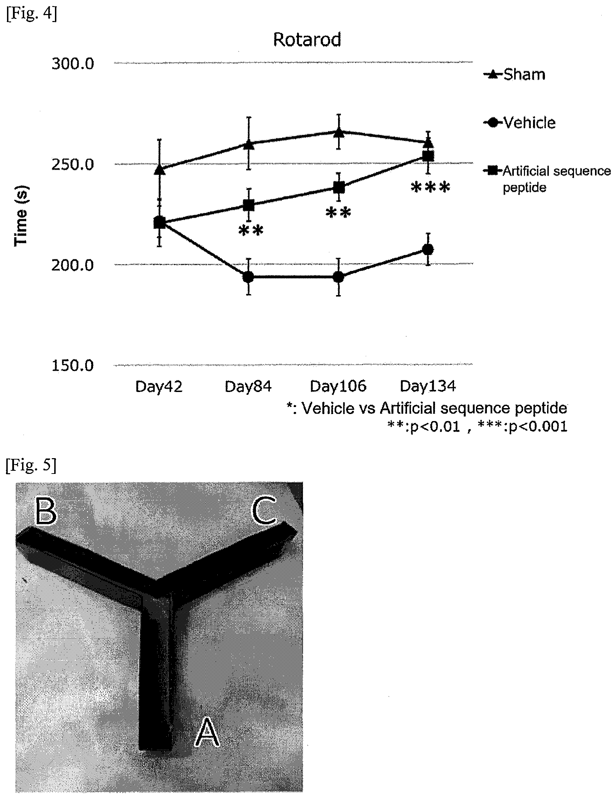

In the beam balance test, significant reductions in the number of slips (*** $p < 0.001$) and latency to traverse the beam (** $p < 0.01$, *** $p < 0.001$) were observed in the peptide-treated group compared to the control group (Figs. 3-1 and 3-2). In the rotarod test, the peptide-treated group showed a significant increase in the time spent on the rotor compared to the control group (** $p < 0.01$, *** $p < 0.001$) (Fig. 4). These results demonstrate the efficacy of the artificial sequence peptide in improving motor function in chronic traumatic brain injury.

Example 4

Evaluation of the Efficacy of Artificial Sequence Peptides for Severe Traumatic Brain Injury

(1) Materials and Methods

i) Preparation of a mouse model of severe traumatic brain injury (TBI)

Traumatic brain injury model mice and sham-operated (Sham) mice were prepared by the same method as described in Example 1, except for the condition that caused the contusion with the impactor (the depth was changed to 2.0 mm). Deeper depth of impactor allows for the creation of more severe traumatic brain injury model mice.

ii) Administration of Peptide

Traumatic brain injury model mice prepared as described above were divided into a peptide-treated group (n = 9) and a control group (n = 9). The peptide (hydrochloride) (1.39 mg/kg, or approximately 0.3 $\mu$mol/kg) was administered via the tail vein (10 mL/kg dose volume) for a total of 16 doses: once daily for five consecutive days starting on day 32 after TBI surgery, and then twice weekly starting the following week. To the control group the same volume of saline was injected via the tail vein at the same schedule as in the peptide-treated group. To sham-operated (Sham) mice (n = 7) saline was also injected via the tail vein, as in the control group.

iii) Evaluation of motor function

Motor function was evaluated by beam balance test and rotarod test as described in Example 3.

iii) Evaluation of memory function

Memory function was evaluated by the Y-maze test. The test was performed using the apparatus shown in Fig. 5, which had three arms of the same size connected in a Y-shape (referred to hereinafter as A, B, and C). The entrance to arm C was blocked with a board to allow walking in only two directions (A, B) and each mouse was trained for 3 minutes. During the actual Y-maze test, the direction in which each mouse entered each arm (from arm A to arm B, from arm A to arm C, and so on) was recorded for 8 minutes. An entry was recorded when the entire body was inside an arm. The number of times that three consecutive entries into different arms were observed was counted (e.g., A→B→C was counted, but A→B→A was not), as was the total number of arm entries. Alternation, which is an index of spatial working memory, was calculated using the following formula:

$$\text{Alternation (\%)} = \frac{\text{Number of alternations (number of three consecutive entries into different arms)}}{\text{Total number of arm entries} - 2} \times 100$$

iv) Statistical processing method

Each measurement was presented as the mean $\pm$ standard error of the mean (mean $\pm$ SEM). Comparisons between the control group and the peptide-treated group were performed using the Mann-Whitney U test for the number of slips and the Student's t-test for the latency to traverse the beam and the time spent on the rotor. p values of $< 0.05$, $< 0.01$, or $< 0.001$ were considered statistically significant.

(2) Result

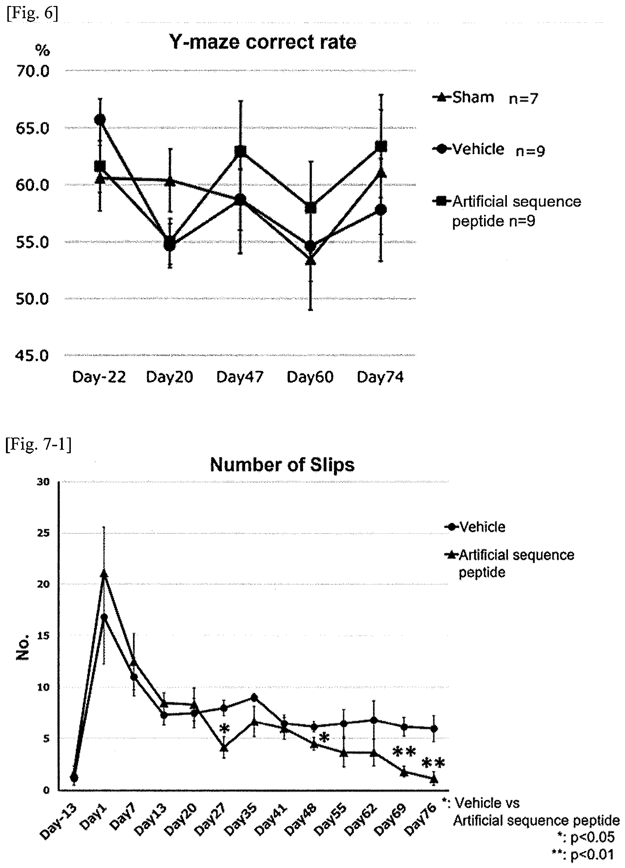

In the beam balance test, the peptide-treated group showed significant reductions in the number of slips and latency to traverse the beam compared to the control group, and in the rotarod test, the peptide-treated group showed a significant increase in the time spent on the rotor compared to the control group (Figure not shown). Furthermore, in the Y-maze test, the peptide-treated group showed a trend toward recovery of memory function compared to the control group (Fig. 6). These results demonstrate the efficacy of the artificial sequence peptide on severe traumatic brain injury.

Example 5

Evaluation of the Efficacy of Artificial Sequence Peptides for Subacute Traumatic Brain Injury

(1) Materials and Methods

i) Production of peptides

An artificial sequence peptide consisting of the amino acid sequence set forth in SEQ ID NO: 3 was chemically synthesized (the resulting peptide was in the form of a hydrochloric acid (HCl) salt).

ii) Preparation of a mouse model of traumatic brain injury

Traumatic brain injury model mice were prepared by the same method as described in Example 1.

iii) Administration of Peptide

Traumatic brain injury model mice prepared as described above were divided into a peptide-treated group (n = 6) and a control group (n = 6). The peptide (hydrochloride) (1.28 mg/kg, or approximately 0.3 μmol/kg) was administered as a single dose via the tail vein (10 mL/kg dose volume) on the 14th day after TBI surgery. To the control group the same volume of saline was injected via the tail vein at the same schedule as in the peptide-treated group.

iv) Evaluation of motor function

Motor function was evaluated by a beam balance test as described in Example 1.

(2) Result

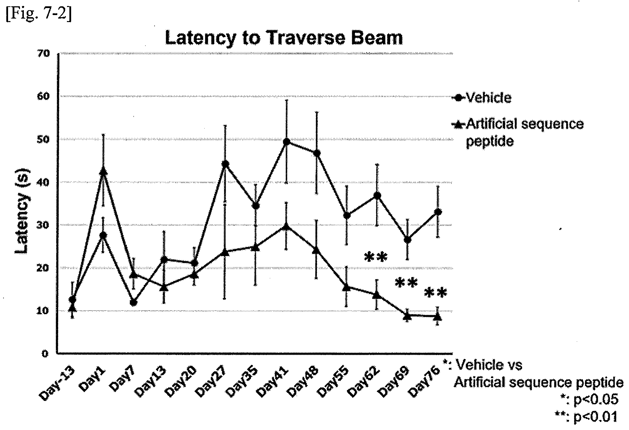

In the beam balance test, the peptide-treated group exhibited significant reductions in the number of slips (* $p <$ 0.05, ** $p < 0.01$) and latency to traverse the beam (* $p < 0.05$, ** $p < 0.01$) compared to the control group (Figs. 7-1 and 7-2). These results demonstrate that the peptide consisting of the amino acid sequence set forth in SEQ ID NO: 3 is effective in improving motor function in subacute traumatic brain injury similarly to the peptide consisting of the amino acid sequence set forth in SEQ ID NO: 1.

Additionally, peptides (in the form of hydrochloride salts) consisting of the amino acid sequences set forth in SEQ ID NOs: 5, 7, 9, 11, 13, 15, 17, 19, 21, 23, 25, and 27 were synthesized and prepared as described above. When these peptides were tested in the same manner as in Example 5, they showed a trend toward improvement in motor function following traumatic brain injury.

INDUSTRIAL APPLICABILITY

The peptide and nucleic acid of the present application can be used to prevent or treat brain injury.

**Claims**

1. A pharmaceutical composition for preventing or treating brain injury, comprising a peptide selected from the following:

   (a) a peptide comprising the amino acid sequence set forth in SEQ ID NO: 1;
   (b) a peptide comprising an amino acid sequence in which one or a plurality of amino acids have been substituted, deleted, inserted, or added in the amino acid sequence set forth in SEQ ID NO: 1; and
   (c) a peptide comprising an amino acid sequence having about 80% or more sequence identity with the amino acid sequence set forth in SEQ ID NO: 1.

2. The pharmaceutical composition according to claim 1, wherein the brain injury is a focal or diffuse brain injury.

3. The pharmaceutical composition according to claim 1, wherein the brain injury is selected from cerebral contusion, epidural hematoma, subdural hematoma, intracerebral hematoma, cerebral concussion, diffuse axonal injury, and subarachnoid hemorrhage.

4. The pharmaceutical composition according to claim 1, wherein the brain injury is traumatic brain injury.

5. A peptide selected from the following:

   (a) a peptide comprising the amino acid sequence set forth in SEQ ID NO: 1;
   (b) a peptide comprising an amino acid sequence in which one or a plurality of amino acids have been substituted, deleted, inserted, or added in the amino acid sequence set forth in SEQ ID NO: 1; and
   (c) a peptide comprising an amino acid sequence having about 80% or more sequence identity with the amino acid sequence set forth in SEQ ID NO: 1.

6. A composition comprising the peptide according to claim 5.

7. A pharmaceutical composition comprising the peptide according to claim 5.

8. A nucleic acid encoding the peptide according to claim 5.

9. A vector comprising the nucleic acid according to claim 8.

10. A host cell comprising the nucleic acid according to claim 8 or the vector according to claim 9.

11. A composition comprising the nucleic acid according to claim 8 or the vector according to claim 9.

12. A pharmaceutical composition comprising the nucleic acid according to claim 8 or the vector according to claim 9.

[Fig. 1-1]

**Number of Slips**

*: Vehicle vs Artificial sequence peptide
* p<0.05
Day 14: p=0.0292

[Fig. 1-2]

**Latency to Traverse Beam**

*: Vehicle vs Artificial sequence peptide
*:p<0.05, **:p<0.01
Day 8 : p = 0.0359
Day 14 : p = 0.0049

[Fig. 2-1]

**Number of Slips**

*: Vehicle vs Artificial sequence peptide

**:p<0.01

EP 4 643 870 A1

[Fig. 2-2]

[Fig. 3-1]

[Fig. 3-2]

Latency to Traverse Beam

[Fig. 4]

Rotarod

Legend: Sham, Vehicle, Artificial sequence peptide

** (Day84), ** (Day106), *** (Day134)

*: Vehicle vs Artificial sequence peptide
**:p<0.01 , ***:p<0.001

[Fig. 5]

[Fig. 6]

**Y-maze correct rate**

[Fig. 7-1]

**Number of Slips**

[Fig. 7-2]

Latency to Traverse Beam

## INTERNATIONAL SEARCH REPORT

| International application No. |
| --- |
| **PCT/JP2023/046633** |

| A. | CLASSIFICATION OF SUBJECT MATTER |
| --- | --- |

*A61K 38/16*(2006.01)i; *A61K 48/00*(2006.01)i; *A61P 25/00*(2006.01)i; *C07K 14/00*(2006.01)i; *C12N 1/15*(2006.01)i; *C12N 1/19*(2006.01)i; *C12N 1/21*(2006.01)i; *C12N 5/10*(2006.01)i; *C12N 15/09*(2006.01)i; *C12N 15/63*(2006.01)i
FI: A61K38/16; C12N15/63 Z; C12N1/15; C12N1/19; C12N1/21; C12N5/10; C07K14/00; A61K48/00; A61P25/00; C12N15/09 Z ZNA

According to International Patent Classification (IPC) or to both national classification and IPC

| B. | FIELDS SEARCHED |
| --- | --- |

Minimum documentation searched (classification system followed by classification symbols)

A61K38/16; A61K48/00; A61P25/00; C07K14/00; C12N1/15; C12N1/19; C12N1/21; C12N5/10; C12N15/09; C12N15/63

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN); GenBank/EMBL/DDBJ/GeneSeq

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | WO 2018/088464 A1 (GENOMIX CO., LTD.) 17 May 2018 (2018-05-17) claims, examples | 1-12 |
| A | WO 2020/071520 A1 (STEMRIM INC.) 09 April 2020 (2020-04-09) claims, examples | 1-12 |
| A | hypothetical protein BGX34_008049 [Mortierella sp. NVP85], GenBank [online], 2020, [retrieved on 05 March 2024], retrieved from the Internet: <URL: https://www.ncbi.nlm.nih.gov/protein/KAF9365838.1/>, accession no. KAF9365838.1 sequence | 1-12 |
| A | MAG: DJ-1/PfpI family protein [Rhodocyclaceae bacterium], GenBank [online], 2021, [retrieved on 05 March 2024], retrieved from the Internet: <URL: https://www.ncbi.nlm.nih.gov/protein/MCB1956679.1>, accession no. MCB1956679.1 sequence | 1-12 |

☐ Further documents are listed in the continuation of Box C.　　☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **05 March 2024** | **12 March 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**

International application No.

**PCT/JP2023/046633**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed.

    b. ☐ furnished subsequent to the international filing date for the purposes of international search (Rule 13*ter*.1(a)),

        ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/JP2023/046633**

| Patent document cited in search report | | | Publication date (day/month/year) | Patent family member(s) | | | Publication date (day/month/year) |
|---|---|---|---|---|---|---|---|
| WO | 2018/088464 | A1 | 17 May 2018 | US | 2019/0358307 | A1 | |
| | | | | claims, examples | | | |
| WO | 2020/071520 | A1 | 09 April 2020 | US | 2022/0009976 | A1 | |
| | | | | claims, examples | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022209604 A

**Non-patent literature cited in the description**

- *Gene*, 1988, vol. 67, 31-40
- *Bio/Technology*, 1988, vol. 6, 47-55
- **MARSHAK et al.** Strategies for Protein Purification and Characterization: A Laboratory Course Manual. Cold Spring Harbor Lab Press, 1996
- Remington's Pharmaceutical Science. Mark Publishing Company